# EUROPÄISCHE PATENTSCHRIFT

(11) **EP 1 374 923 B1**
(45) Veröffentlichungstag und Bekanntmachung des Hinweises auf die Patenterteilung: **30.08.2006**
(21) Anmeldenummer: 03011967.1
(22) Anmeldetag: 28.05.2003
(51) Int. Cl.: A61L 27/54, A61L 27/56

(54) **Poröse Implantatkörper mit antibiotischer Beschichtung, ihre Herstellung sowie Verwendung**
Porous implants with antibiotic coating, their preparation and use
Implants poreux avec un revêtement antibiotique, leur préparation et utilisation

(30) Priorität: 21.06.2002 DE 10227935
(43) Veröffentlichungstag der Anmeldung: 02.01.2004
(73) Patentinhaber: Heraeus Kulzer GmbH, 63450 Hanau (DE)
(72) Erfinder: Vogt, Sebastian, Dr., 99084 Erfurt (DE); Schnabelrauch, Matthias, Dr., 07749 Jena (DE); Kühn, Klaus-Dieter, Dr., 35041 Marburg (DE)
(74) Vertreter: Kühn, Hans-Christian

(56) Entgegenhaltungen:
- EP-A- 1 287 818
- DE-A- 3 248 328
- US-A- 5 679 646

## Beschreibung

Die vorliegende Erfindung betrifft eine antibiotische Beschichtung von (interkonnektierend) porösen Körpern und Verfahren zu ihrer Herstellung sowie ihre Verwendung. Diese antibiotisch ausgerüsteten porösen Körper sollen als Implantate in der Human- und Veterinärmedizin zur Behandlung von Knochendefekten und gegebenenfalls zur Behandlung von Weichteildefekten Verwendung finden. Dabei wird eine kontinuierliche Antibiotika-Freisetzung aus der auf der inneren Oberfläche der Porensysteme befindlichen antibiotischen Beschichtung über einen Zeitraum von mehreren Tagen angestrebt, damit eine bakterielle Infektion im Bereich des zu behandelnden Knochendefekts und/oder Weichteildefektes wirksam verhindert oder bekämpft werden kann. Insbesondere sollen solche bakteriellen Erreger behandelt werden, die Resistenzen gegenüber den üblich verwendeten Antibiotika ausgebildet haben.

Knochendefekte treten in der Human- und Veterinärmedizin relativ häufig auf und werden insbesondere durch Knochenfisteln, Trümmerfrakturen und Tumoren verursacht. Bei offenen Trümmerfrakturen werden vielfach zusätzlich Infektionen des Knochengewebes beobachtet. Die Behandlung von Knochendefekten kann durch Auffüllung mit geeigneten Implantaten erfolgen. In den letzten Jahren haben insbesondere poröse Implantate Interesse gefunden, die auf Grund ihrer chemischen Zusammensetzung und ihrer Porosität eine osteokonduktive Wirkung aufweisen und ein Einwachsen des umgebenden Knochengewebes begünstigen. Problematisch ist die Behandlung von Knochendefekten immer dann, wenn zusätzlich mikrobielle Infektionen des Knochengewebes vorhanden sind. Infektionen des Knochengewebes können nach vorheriger chirurgischer Sanierung durch systemische oder lokale Applikation von geeigneten Antibiotika bekämpft werden. Die systemische Anwendung von Antibiotika ist auf Grund der mitunter nicht unbeträchtlichen Toxizität der Antibiotika problematisch. Die lokale Applikation direkt im oder am infizierten Gewebe, nach entsprechender chirurgischer Sanierung, bietet dagegen den Vorteil, dass hohe lokale Antibiotika-Konzentrationen erreicht werden können unter Vermeidung von schädigenden Antibiotika-Konzentrationen im übrigen Organismus. Durch diese hohen lokalen Antibiotika-Konzentrationen am Ort der bakteriellen Infektion ist eine weitgehende Abtötung der Mikroorganismen möglich, so dass die bakteriellen Infektionen sehr wirksam behandelt werden. Besonders vorteilhaft ist es, wenn am Ort der bakteriellen Infektionen eine wirksame Antibiotikum-Konzentration über einen Zeitraum von mehreren Tagen bis Wochen aufrecht erhalten wird, damit das Antibiotikum möglichst tief in das infizierte Gewebe eindringen kann und dadurch auch schwer zugängliche Keime abgetötet werden. Weichteildefekte mit bakteriellen Infektionen sind in der Human- und Veterinärmedizin ebenfalls häufig zu finden. Die lokale Antibiotika-Applikation ist auch zur Behandlung dieser Infektionen von Interesse.

Bisher fanden schwerlösliche Salze der Aminoglykosid-Antibiotika und der Lincosamid-Antibiotika relativ geringe Beachtung für die Herstellung von Depotpräparaten und von antibiotisch wirksamen Implantaten. Bei den Aminoglykosid-Antibiotika sind einige wenige geringlösliche Salzen bekannt. So wurde beim Gentamicin die Darstellung geringlöslicher Salze basierend auf höheren Fettsäuren, Arylalkylcarbonsäuren, Alkylsulfaten und Alkylsulfonaten beschrieben (G. M. Luedemann, M. J. Weinstein: Gentamycin and method of production. 16.07.1962, **US 3,091,572**). Exemplarisch sind dafür Gentamicin-Salze der Laurinsäure, der Stearinsäure, der Palmitinsäure, der Ölsäure, der Phenylbuttersäure, der Naphthalen-1-carbonsäure. Die Synthese der Dodecylsulfate des Gentamicins in wäßriger beziehungsweise wäßrig-methanolischer Lösung sind von Jurado Soler et al. beschrieben worden (A. Jurado Soler, J. A. Ortiz Hernandez, C. Ciuro Bertran: Neue Gentamicinderivate, Verfahren zur Herstellung derselben und diese enthaltende antibiotisch wirksame Zusammensetzung. 30.09.1974 **DE 24 46 640**). Diese Salze erwiesen sich jedoch vielfach als unvorteilhaft, weil sie wachsartige, hydrophobe Substanzen darstellen, die eine galenische Verwendung behindern. Weiterhin wurden Festtsäuresalze und aliphatische Sulfate von Gentamicin und von Etamycin aus der freien Base beziehungsweise aus ihren Salzen in Wasser bei 50-80°C synthetisiert (H. Voege, P. Stadler, H. J. Zeiler, S. Samaan, K. G. Metzger: Schwerlösliche Salze von Aminoglykosiden sowie diese enthaltende Formulierungen mit verzögerter Wirkstoff-Freigabe. 28.12.1982 **DE 32 48 328**). Diese Antibiotika-Fettsäuresalze sollten als Injektionspräparate geeignet sein. Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, **US 4,617,293**). Es werden die Salze der Phosphorsäuremonoester von Derivaten der Hydroxyflavane, Hydroxyflavene, Hydroxyflavanone, Hydroxyflavone und Hydroxyflavylium beschrieben. Besonders bevorzugt sind dabei die Derivate der Flavanone und der Flavone. Diese schwerlöslichen Salze sollen als Depotpräparate Verwendung finden. So werden zum Beispiel diese Salze in Kollagenvliese eingebracht (H. Wahlig, E. Dingeldein, D. Braun: Medicinally useful, shaped mass of collagen resorbable in the body. 22.09.1981, **US 4,291,013**). Weiterhin wurden auch künstliche Herzklappen mit diesen schwerlöslichen Gentamicin-Salzen, Gentamicin Crobefat, imprägniert (M. Cimbollek, B.Nies, R. Wenz, J. Kreuter: Antibioticimpregnated heart valve sewing rings for treatment and prophylaxis of bacterial endocarditis. Antimicrob. Agents Chemother. 40(6) (1996)1432-1437).

Die Erzeugung von einfachen Antibiotikum-/Antibiotka-Depots in den Porensystemen von porösen Körpern durch Tränken von porösen Körpern mit wässrigen Antibiotika-Lösungen ist allgemeiner Kenntnisstand (R. Reiner, W. Kißing, H. Döring, K. Köster, H. Heide: Implantierbares Pharmaka-Depot. 20.02.1978 **DE 28 07 132**). Hierbei kann eine retardierende Wirkstoffreisetzung des in Wasser löslichen Wirkstoffs nur durch Adsorptions- und/oder durch Diffusionsprozesse erreicht werden, die vom verwendeten Material, dem Porenvolumen und der Porosität abhängt. Daneben ist es auch möglich, in Wasser gering lösliche Antibiotika-Salze in geeigneten organischen Lösungsmitteln zu lösen und mit diesen Lösungen die Formkörper zu tränken. Dadurch entstehen Wirkstoffdepots in den Formkörpern, die eine retardierende Wirkstofffreisetzung zeigen. Ein Beispiel dafür ist die von Cimbollek und Nies beschriebene Methode zur Lösung eines in Wasser gering löslichen Gentamicin-Salzes und deren Verwendung zur Beschichtung (M. Cimbollek, B. Nies: Solvent for a sparinly soluble gentamicin salt. 04.05.1994 **US 5,679,646**). Dieses Gentamicinsalz auf Basis von 3-p-Methoxybezylidene-6-hydroxy-4'-methoxy-flavanone-6-phosphat muss jedoch vor der Beschichtung synthetisiert werden. Von Kurtz wird eine interessante Variante beschrieben, bei der in Wasser gering lösliche Antibiotika-Salze in situ in einer nicht näher spezifizierten Unterlage durch aufeinanderfolgende Tränkung mit einer Lösung eines basischen Gentamicin-Salzes beziehungsweise eines Polymycin-Salzes und eines sauren Penicillin- beziehungsweise Cephalosporinsalzes gebildet werden (L. D. Kurtz: Wasserunlösliche biocide Antibiotikasalze. 13.11.1973 **DE 23 01 633**). Die Penicillin- bzw. Cephalosporinreste bilden die anionische Komponente der Salze und die kationischen Aminoglukosid-Reste die kationische Komponente.

Dieses interessante Konzept der Beschichtung wurde später nicht wieder aufgegriffen und auch nicht auf seine Eignung für andere in Wasser gering lösliche Salze der Aminoglykosid-Antibiotika, der Lincosamid-Antibiotika und der Glykopeptid-Antibiotika geprüft. Bisher sind keine ähnlichen Imprägnierungsverfahren zur Erzeugung von Antibiotika-Depots in porösen Körpern unter Nutzung von anionischen Resten aus den Gruppen der Fettsäuresalze bekannt.

Die Schichtbildungseigenschaften von in Wasser gering löslichen Antibiotika-Salzen auf Basis von Fettsäuresalzen und Alkylsulfaten fanden ebenfalls bisher keine Beachtung.

Zusammenfassend kann festgestellt werden, dass bisher keine Verfahren bekannt sind, bei denen antibiotische Beschichtungen auf der Oberfläche von interkonnektierenden Porensystemen aufgebracht werden, die aus in Wasser gering löslichen organischen Salzen des Netilmicins, des Sisomicins, des Kanamycins, des Amikacins, des Vancomycins und des Clindamicins bestehen und die direkt in den zu beschichtenden Porensystemen ausgehend von in Wasser löslichen Salzen des Netilmicins, des Sisomicins, des Kanamycins, des Amikacins, des Vancomycins sowie des Clindamicins und von in Wasser löslichen Fettsäuresalzen und/oder Alkylsulfaten synthetisiert werden.

Der Erfindung liegt die Aufgabe zu Grunde, eine antibiotische Beschichtung von porösen Körpern zu entwickeln, die im wäßrigen Milieu Antibiotika verzögert über einen Zeitraum von mehreren Tagen bis zu wenigen Wochen kontinuierlich freisetzt.

Die Aufgabe wird erfindungsgemäß durch die Merkmale der unabhängigen Ansprüche gelöst. Vorteilhafte Ausführungen sind in den Unteransprüchen angegeben.

Der Erfindung liegt der überraschende Befund zu Grunde, dass die Laurate und die Dodecylsulfate des Netilmicins und des Sisomicins in Wasser gering löslich sind und auf Grund ihrer klebrigen Konsistenz auf nichtmetallischen und metallischen Oberflächen haften, ohne dass polymere Schichtbildner notwendig sind. Bei diesen Salzen stellen das Netilmicin und das Sisomicin die kationische Komponente der Antibiotika-Salze dar und die Laurat-Reste und die Dodecylsulfat-Reste die anionische Komponente. Diese Salze lösen sich im wäßrigen Milieu langsam unter Freisetzung von Netilmicin und/oder Sisomicin auf. Im folgenden werden unter dem Begriff der Netilmicin-Laurate die Mono-, Di-, Tri- und Tetra-Laurate des Netilmicins verstanden. Unter dem Begriff der Sisomicin-Laurate werden die Mono-, Di-, Tri- und Tetra-Laurate des Sisomicins verstanden. In gleicher Weise werden die Myristate dieser Antibiotika verstanden. DieLaurate des Netilmicins und des Sisomicins lösen sich vorteilhaft in Methanol und Ethanol. Die Beschichtung haftet ohne polymere Bindemittel auf der inneren Oberfläche von Porensystemen von porösen Körpern und löst sich im wäßrigen Milieu unter kontinuierlicher Antibiotika-Freisetzung vollständig auf.

Der Erfindung liegt weiterhin der überraschende Befund zu Grunde, dass Clindamycin mit Laurinsäure gering lösliche Salze bildet, die in Porensystemen Beschichtungen ausbilden können. Diese Salze lösen sich langsam unter Freisetzung von Clindamycin auf.

Überraschenderweise sind das Laurat, das Myristat und das Dodecylsulfat des Vancomycins ebenfalls in Wasser gering löslich. Diese Salze eignen sich auch zur Herstellung von Beschichtungen und setzen das Vancomycin im wäßrigen Milieu retardiert frei.

Der Erfindung liegt weiterhin der überraschende Befund zu Grunde, das in Wasser lösliche Amikacin-Salze und Kanamycin-Salze mit in Wasser löslichen Salzen der Laurinsäure, der Myristinsäure und der Dodecylschwefelsäure in Wasser gering lösliche Salze bilden. Unter dem Begriff des Amikacin-Laurates werden die Mono-, Di-, Tri- und Tetra-Laurate des Amikacins verstanden. In gleicher Weise wird der Begriff Kanamycin-Laurat verwendet. Auch die Begriffe Amikacin-Myristat und Kanamycin-Myristat umfassen die Mono-, Di-, Tri- und Tetra-Myristate dieser Antibiotika. Amikacin-Laurat und Kanamycin-Laurat lösen sich partiell in Gemischen aus Dioxan und Wasser und auch in Gemischen aus Tetrahydrofuran und Wasser. Das Amikacin-Laurat, das Amikacin-Myristat, das Amikacin-Dodecylsulfat, das Kanamycin-Laurat, das Kanamycin-Myristat und das Kanamycin-Dodecylsulfat setzen im wäßrigen Milieu die zu Grunde liegenden Antibiotika verzögert frei.

Überraschenderweise besitzen die Gentamicinsalze der Laurinsäure und der Myristinsäure eine wachsartige Konsistenz und haften sehr gut auf unterschiedlichsten nichtmetallischen und metallischen Oberflächen. Auch diese Salze setzen unter Auflösung Gentamicin verzögert frei. Unter dem Begriff des Gentamicin-Laurates werden die Mono-, Di-, Tri-, Tetra- und Penta-Laurate des Gentamicins verstanden. In gleicher Weise wird der Begriff Gentamicin-Myristat verwendet.

Es wurde überraschend gefunden, dass das Laurat, das Myristat und das Dodecylsulfat des Vancomycins in Wasser gering löslich sind und Schichten auf Oberflächen bilden können. Diese Salze setzen Vancomycin im wäßrigen Milieu verzögert frei.

Das Laurat, das Myristat und das Dodecylsulfat des Tobramycins sind ebenfalls in Wasser gering löslich und können zur Schichtbildung verwendet werden.

Es ist erfindungsgemäß, dass eine Beschichtung aus mindestens einem in Wasser oder im wäßrigen Milieu gering löslichen Antibiotika-Salz aus der Gruppe des Netilmicin-Laurates, des Netilmicin-Myristates, des Netilmicin-Dodecylsulfates, des Sisomicin-Laurates, des Sisomycin-Myristates, des Sisomicin-Dodecylsulfates, des Gentamicin-Laurates, des Gentamicin-Myristates, des Clindamycin-Laurates, des Amikacin-Laurates, des Amikacin-Myristates, des Amikacin-Dodecylsulfates, des Kanamycin-Laurates, des Kanamycin-Myristates, des Kanamycin-Dodecylsulfates, des Tobramycin-Laurates, des Tobramycin-Myristates, des Tobramycin-Dodecylsulfates, des Vancomycin-Laurates, des Vancomycin-Myristates und des Vancomycin-Dodecylsulfates in das Porensystem von **porösen medizinischen Implantat-Körpern** eingebracht ist.

Weiterhin ist erfindungsgemäß, dass zuerst eine wäßrige Lösung, die mindestens einen Vertreters eines in Wasser leicht löslichen Salzes des Netilmicins, des Sisomicins, des Clindamycins, des Amikacins, des Kanamycins, des Tobramycins und des Vancomycins enthält, in das Porensystem der porösen Körper eingebracht wird und dass nach einem Trocknungsschritt eine zweite wäßrige Lösung eines in Wasser leicht löslichen Salzes der Laurinsäure, Myristinsäure und/oder Dodecylschwefelsäure eingebracht wird und dabei eine in Wasser gering lösliche antibiotische Beschichtung im Porensystem des porösen Körpers ausbildet wird.

Im Sinne der Erfindung ist, dass die Reihenfolge der Beschichtungsschritte vertauscht werden kann.

Es ist des weiteren erfindungsgemäß, dass eine methanolische Lösung oder eine ethanolische Lösung mindestens eines Vertreters des Netilmicin-Laurates, des Netilmicin-Dodecylsulfates, des Sisomicin-Laurates, des Sisomicin-Dodecylsulfates, des Gentamicin-Laurates, des Vancomycin-Laurates, des Vancomycin-Dodecylsulfates und des Clindamycin-Laurates in das Porensystem des porösen Körpers eingebracht wird und dass durch Verdampfung oder Verdunstung des Methanols oder des Ethanols eine in Wasser gering lösliche antibiotische Beschichtung gebildet wird.

Außerdem ist erfindungsgemäß, dass in einem Gemisch von Dioxan-Wasser und/oder Tetrahydrofuran/Wasser Amikacin-Laurat, Kanamycin-Laurat, Amikacin-Dodecylsulfat und Kanamycin-Dodecylsulfat partiell gelöst und/oder suspendiert wird und diese Lösungen und/oder Suspensionen in das Porensystem der porösen Körper eingebracht werden und dass durch Verdampfung oder Verdunstung der Gemische aus Dioxan und Wasser oder der Gemische aus Tetrahydrofuran und Wasser eine in Wasser gering lösliche antibiotische Beschichtung gebildet wird.

Es ist zweckmäßig, dass die antibiotische Beschichtung auf poröse medizinische Implantat-Körper, die in Form von porösen Pulvern, porösen Granulaten, porösen Formkörpern und/oder porösen Schichten auf kompakten Körpern vorliegen, aufgebracht wird.

Vorteilhaft ist, dass die Beschichtung von porösen medizinischen Implantat-Körpern, die vorzugsweise die Form von porösen Pulvern und/oder porösen Granulaten aufweisen, durch Zusatz von mindestens einem in Wasser oder im wäßrigen Milieu gering löslichen Antibiotika-Salz aus der Gruppe des Netilmicin-Laurates, des Netilmicin-Myristates, des Netilmicin-Dodecylsulfates, des Sisomicin-Laurates, des Sisomicin-Myristates, des Sisomicin-Dodecylsulfates, des Gentamicin-Laurates, des Gentamicin-Myristates, des Clindamycin-Laurates, des Amikacin-Laurates, des Amikacin-Dodecylsulfates, des Kanamycin-Laurates, des Kanamycin-Dodecylsulfates, des Tobramycin-Laurates, des Tobramycin-Myristates, des Tobramycindodecylsulfates, des Vancomycin-Laurates, des Vancomycin-Myristates und des Vancomycin-Dodecylsulfates insbesondere durch Vermahlung, gegebenenfalls unter Zusatz von Methanol, Ethanol, Dioxan, Tetrahydrofuran und/oder Wasser oder deren Gemischen gebildet ist.

Es ist vorteilhaft, dass die Beschichtung von porösen medizinischen Implantat-Körpern, die vorzugsweise die Form von porösen Pulvern und/oder porösen Granulaten aufweisen, durch Zusatz, insbesondere durch Vermahlung dieser Pulver und/oder Granulate mit einem Gemisch aus mindestens einem in Wasser löslichen Salz des Netilmicins, des Sisomicins, des Clindamycins, des Amikacins, des Kanamycins, des Vancomycins und mindestens einem in Wasser löslichen Salzes der Laurinsäure und/öder Myristinsäure und/oder Dodecylschwefelsäure in Gegenwart von Wasser oder wäßrigen Lösungen vorgenommen wird.

Es ist auch zweckmäßig, dass die Beschichtung gegebenenfalls zusätzlich in Wasser leicht lösliche Salze des Gentamicins, des Netilmicins, des Sisomicins, des Amikacins, des Kanamycins, des Clindamycins, des Tobramycins, des Vancomycins, des Ciprofloxacins und/oder des Moxifloxacins enthält.

Vorteilhafterweise wird die antibiotische Beschichtung auf resorbierbare, auf partiell resorbierbare und/oder auf nicht resorbierbare, biokompatible, poröse medizinische Implantat-Körper aufgebracht.

Gegenstand der Erfindung ist weiterhin, dass die antibiotisch beschichteten porösen medizinischen Implantat-Körper in Form von beschichteten porösen Granulaten und/oder beschichteten porösen Pulvern zu Formkörpern verpresst werden, die als Implantate verwendet werden.

Es ist erfindungsgemäß, dass die antibiotisch beschichteten porösen Granulate und/oder antibiotisch beschichteten porösen Pulver als Bindemittel für die Herstellung von Formkörpern durch Verpressung von Pulvergemischen verwendet werden.

Es ist auch erfindungsgemäß, dass die antibiotisch beschichteten porösen medizinischen Implantat-Körper als temporäre und/oder als dauerhafte Implante verwendet werden.

Wesentlich ist für die Erfindung die Verwendung von in Wasser gering löslichen Salzen aus der Gruppe des Netilmicin-Laurates, des Netilmicin-Myristates, des Netilmicin-Dodecylsulfates, des Sisomicin-Laurates, des Sisomicin-Myristates, des Sisomicin-Dodecylsulfates, des Amikacin-Laurates, des Amikacin-Myristates, des Amikacin-Dodecylsulfates, des Kanamycin-Laurates, des Kanamycin-Myristates, des Kanamycin-Dodecylsulfates, des Vancomycin-Dodecylsulfates, des Vancomycin-Laurates, des Vancomycin-Myristates, des Ciprofloxacin-Myristates und des Clindamicin-Laurates als Antibiotikum-/Antibiotika-Depot von Implantaten.

Die Erfindung soll nachstehend an den Beispielen 1-3 erläutert werden, ohne die Erfindung einzuschränken.

Als Körper mit interkonnektierenden Porensystemen wurden quaderförmige, resorbierbare Phosphatgläser mit den Abmessungen von 20x20x10 mm für die Beispiele 1-3 verwendet. Diese Körper hatten eine Gesamt-Porosität von 65 Volumenprozent. Davon waren 50 Prozent Makroporen (250-300µm Porendurchmesser) und 15 Prozent Mikroporen (Porendurchmesser < 100µm).

### Beispiel 1:

Es wurden 80 mg Gentamicinsulfat (AK628) in 1,92 g bidest. Wasser gelöst (Lösung 1). Separat wurden 75 mg Natriumlaurat in 1 g eines 50 %igen Wasser-Ethanol-Gemischs gelöst (Lösung 2). In die Poren der quaderförmigen Phosphatgläser wurden zuerst die zuvor hergestellten Lösung 1 getropft. Die Probekörper saugten die Lösung 1 auf. Danach wurde das in den Poren befindliche Wasser durch Trocknen über wasserfreiem Calciumchlorid entfernt. Dann wurden die vorbereiteten Lösung 2 in die Poren der getrockneten Phosphatgläser getropft. Die Trocknung der Probekörper erfolgte danach ebenfalls über wasserfreiem Calciumchlorid bis zur Massekonstanz.

### Beispiel 2:

Es wurden 80 mg Clindamycinhydrochlorid in 1,92 g bidest. Wasser gelöst (Lösung 1). Separat wurden 20 mg Natriumlaurat in 1 g eines Wasser gelöst (Lösung 2). In die Poren der quaderförmigen Phosphatgläser wurden zuerst die zuvor hergestellten Lösung 1 getropft. Die Probekörper saugten die Lösung 1 auf. Danach wurde das in den Poren befindliche Wasser durch Trocknen über wasserfreiem Calciumchlorid entfernt. Dann wurden die vorbereiteten Lösung 2 in die Poren der getrockneten Phosphatgläser getropft. Die Trocknung der Probekörper erfolgte danach ebenfalls über wasserfreiem Calciumchlorid bis zur Massekonstanz.

### Beispiel 3:

Es wurden 80 mg Kanamycinsulfat in 1,92 g bidest. Wasser gelöst (Lösung 1). Separat wurden 93 mg Natriumlaurat in 1 g eines 50 %igen Wasser-Ethanol-Gemischs gelöst (Lösung 2). In die Poren der quaderförmigen Phosphatgläser wurden zuerst die zuvor hergestellten Lösung 1 getropft. Die Probekörper saugten die Lösung 1 auf. Danach wurde das in den Poren befindliche Wasser durch Trocknen über wasserfreiem Calciumchlorid entfernt. Dann wurden die vorbereiteten Lösung 2 in die Poren der getrockneten Phosphatgläser getropft. Die Trocknung der Probekörper erfolgte danach ebenfalls über wasserfreiem Calciumchlorid bis zur Massekonstanz.

Die Massen der eingebrachten Antibiotika und des Natriumlaurates wurden gravimetrisch bestimmt.

**Tabelle 1:**

| | Masse des eingebrachten Antibiotikums [mg] | Masse des eingebrachten Natriumlaurats [mg] |
|---|---|---|
| Beispiel 1 | 59,1 | 72,0 |
| Beispiel 2 | 58,6 | 18,3 |
| Beispiel 3 | 61,8 | 91,0 |

Die in den Beispielen 1-3 beschichteten Formkörper wurden in jeweils 10 ml Sörensen-Puffer pH 7,4 eingebracht und in dieser bei 37°C über einen Zeitraum von 12 Tagen gelagert. Die Probennahme erfolgte täglich. Nach jeder Probennahme wurde das Freisetzungsmedium vollständig durch frisches Medium ersetzt. Die Freisetzung der Antibiotika wurden mit einem Agardiffusionstest unter Verwendung von Bacillus subtilis ATCC 6633 als Testkeim erfasst und die Hemmhofdurchmesser gescannt und anschließend mit Hilfe einer speziellen Software ausgemessen. Die Ergebnisse sind in Tabelle 2 dargestellt. Beim Beispiel 1 wurde mit Hilfe von Gentamicin-Standards die jeweils freigesetzte Gentamicinmenge quantitativ bestimmt.

**Tabelle 2:**

| | ■ | **Antibiotika-Freisetzung** | | | | |
|---|---|---|---|---|---|---|
| | Beispiel 1 | | Beispiel 2 | | Beispiel 3 | |
| Zeit [d] | Verdünnung | Hemmhofdurchmesser [mm] | Verdünnung | Hemmhofdurchmesser [mm] | Verdünnung | Hemmhofdurchmesser [mm] |
| 1 | 1:250 | 20,05 | 1:100 | 20,30 | 1:100 | 21,00 |
| 2 | 1:100 | 17,10 | 1:20 | 18,65 | 1:40 | 21,50 |
| 3 | 1:40 | 19,43 | 1:10 | 18,73 | 1:10 | 22,00 |
| 6 | 1:5 | 20,20 | unverdünnt | 15,13 | 1:5 | 22,20 |
| 9 | unverdünnt | 22,50 | unverdünnt | 0,00 | 1:3 | 19,95 |
| 12 | unverdünnt | 23,35 | unverdünnt | 0,00 | unverdünnt | 23,00 |

**Tabelle 3:**

| ■ | **Zeit [d]** | 1 | 2 | 3 | 6 | 9 | 12 |
|---|---|---|---|---|---|---|---|
| Gentamicinfreisetzung (als Gentamicinbase) [mg] | | 16,28 | 2,97 | 1,65 | 0,19 | 0,09 | 0,11 |

## Patentansprüche

1. Poröse medizinische Implantat-Körper mit antibiotischer Beschichtung, **dadurch gekennzeichnet, dass** eine Beschichtung aus mindestens einem in Wasser oder im wäßrigen Milieu gering löslichen Antibiotika-Salz aus der Gruppe des Netilmicin-Laurates, des Netilmicin-Dodecylsulfates, des Netilmicin-Myristates, des Sisomicin-Laurates, des Sisomicin-Myristates, der Sisomicin-Dodecylsulfates, des Gentamicin-Laurates, des Gentamicin-Myristates, des Clindamycin-Laurates, des Amikacin-Laurates, des Amikacin-Myristates, des Amikacin-Dodecylsulfates, des Kanamycin-Laurates, des Kanamycin-Myristates, des Kanamycin-Dodecylsulfates, des Vancomycin-Laurates, des Vancomycin-Dodecylsulfates, des Vancomycin-Myristates, des des Tobramycin-Laurates, des Tobramycin-Myristates, des Tobramycin-Dodecylsulfates, des Ciprofloxacin-Laurates, des Ciprofloxacin-Myristates in das Porensystem von nichtmetallischen porösen Körpern und/oder von metallischen porösen Körpern eingebracht ist.

2. Verfahren zur Herstellung von porösen medizinischen Implantat-Körpern mit einer antibiotischen Beschichtung nach Anspruch 1, **dadurch gekennzeichnet, dass** zuerst eine wäßrige Lösung, die mindestens einen Vertreter eines in Wasser leicht löslichen Salzes des Netilmicins, des Sisomicins, des Clindamycins, des Amikacins, des Kanamycins, des Tobramycins, des Vancomycins, des Ciprolfoxacins enthält, in das Porensystem von porösen Körpern eingebracht wird und dass nach einem Trocknungsschritt eine zweite wäßrige Lösung eines in Wasser leicht löslichen Salzes der Laurinsäure, der Myristinsäure und/oder der Dodecylschwefelsäure eingebracht wird und dabei eine in Wasser gering lösliche antibiotische Beschichtung im Porensystem des porösen Körpers ausbildet wird.

3. Verfahren nach Anspruch 2, **dadurch gekennzeichnet, dass** die Reihenfolge der Beschichtungsschritte vertauscht wird.

4. Verfahren zur Herstellung von porösen medizinischen Implantat-Körpern mit einer antibiotischen Beschichtung nach Anspruch 1, **dadurch gekennzeichnet, dass** eine methanolische Lösung oder eine ethanolische Lösung mindestens eines Vertreters aus der Gruppe des Netilmicin-Laurates, des Netilmicin-Myristates, des Netilmicin-Dodecylsulfates, des Sisomicin-Laurates, des Sisomicin-Myristates, des Sisomicin-Dodecylsulfates, des Gentamicin-Laurates, des Gentamicin-Myristates, des Clindamycin-Laurates, des Tobramycin-Laurates, des Tobramycin-Myristates, des Tobramycin-Dodecylsulfates, des Ciprofloxacin-Myristates, des Vancomycin-Laurates, des Vancomycin-Myristates, und des Vancomycin-Dodecylsulfates, in das Porensystem des porösen Körpers eingebracht wird und dass durch Verdampfung oder Verdunstung des Methanols oder des Ethanols eine in Wasser gering lösliche antibiotische Beschichtung gebildet wird.

5. Verfahren zur Herstellung von porösen medizinischen Implantat-Körpern mit einer antibiotischen Beschichtung nach Anspruch 1, **dadurch gekennzeichnet, dass** in einem Gemisch von Dioxan-Wasser und/oder Tetrahydrofuran-Wasser Amikacin-Laurat, Kanamycin-Laurat, Amikacin-Dodecylsulfat und/oder Kanamycin-Dodecylsulfat partiell gelöst und/oder suspendiert wird und diese Lösungen und/oder Suspensionen in das Porensystem der porösen Körper eingebracht werden und dass durch Verdampfung oder Verdunstung der Gemische aus Dioxan und Wasser oder der Gemische aus Tetrahydrofuran und Wasser eine in Wasser gering lösliche antibiotische Beschichtung gebildet wird.

6. Poröse medizinische Implantat-Körper mit antibiotischer Beschichtung nach Anspruch 1, **dadurch gekennzeichnet, dass** die antibiotische Beschichtung auf poröse Körper, die in Form von porösen Pulvern, porösen Granulaten, porösen Formkörpern und/oder porösen Schichten auf kompakten Körpern vorliegen, aufgebracht ist.

7. Poröse medizinische Implantat-Körper mit antibiotischer Beschichtung nach Anspruch 1, **dadurch gekennzeichnet, dass** die Beschichtung von porösen Körpern, die vorzugsweise die Form von porösen Pulvern und/oder porösen Granulaten aufweisen, durch Zusatz von mindestens einem in Wasser oder im wäßrigen Milieu gering löslichen Antibiotika-Salz aus der Gruppe des Netilmicin-Laurates, des Netilmicin-Myristates, des Netilmicin-Dodecylsulfates, des Sisomicin-Laurates, des Sisomicin-Myristates, des Sisomicin-Dodecylsulfates, des Gentamicin-Laurates, des Gentamicin-Myristates, des Clindamycin-Laurates, des Amikacin-Laurates, des Amikacin-Myristates, des Amikacin-Dodecylsulfates, des Kanamycin-Laurates, des Kanamycin-Myristates, des Kanamycin-Dodecylsulfates, des Ciprofloxacin-Myristates, des Tobramycin-Laurates, des Tobramycin-Myristates, des Tobramycin-Dodecylsulfates, des Vacomycin-Laurates, des Vancomycin-Myristates, und des Vancomycin-Dodecylsulfates, insbesondere durch Vermahlen, unter Zusatz von Methanol, Ethanol, Dioxan, Tetrahydrofuran und/oder Wasser oder deren Gemische gebildet ist.

8. Poröse medizinische Implantat-Körper mit antibiotischer Beschichtung nach Anspruch 1, **dadurch gekennzeichnet, dass** die Beschichtung von porösen Körpern, die vorzugsweise die Form von porösen Pulvern und/oder porösen Granulaten aufweisen, durch Zusatz von einem Gemisch aus mindestens einem in Wasser löslichen Salz des Netilmicins, des Sisomicins, des Clindamycins, des Amikacins, des Kanamycins, des Tobramycins, des Vancomycins und/oder des Ciprofloxacins und mindestens einem in Wasser löslichen Salzes der Laurinsäure, der Myristinsäure und/oder Dodecylschwefelsäure in Gegenwart von Wasser oder wäßrigen Lösungen, insbesondere durch Vermahlen, gebildet ist.

9. Poröse medizinische Implantat-Körper mit antibiotischer Beschichtung nach Anspruch 1, **dadurch gekennzeichnet, dass** die Beschichtung gegebenenfalls zusätzlich in Wasser leicht lösliche Salze des Gentamicins, des Netilmicins, des Sisomicins, des Amikacins, des Kanamycins, des Clindamycins, des Tobramycins, des Vancomycins, des Ciprofloxacins und/oder des Moxifloxacins enthält.

10. Poröse medizinische Implantat-Körper mit antibiotischer Beschichtung_nach Anspruch 1, **dadurch gekennzeichnet, dass** die antibiotische Beschichtung auf resorbierbare, poröse Körper, auf partiell resorbierbare poröse Körper und/oder auf nicht resorbierbare, biokompatible, poröse Körper aufgebracht ist.

11. Verwendung von porösen medizinische Implantat-Körpern mit antibiotischer Beschichtung nach Anspruch 1, die als antibiotisch beschichtete poröse Granulate und/oder antibiotisch beschichtete poröse Pulver ausgebildet sind, als Bindemittel für die Herstellung von Formkörpern durch Verpressung von Pulvergemischen.

12. Poröse medizinische Implantat-Körper mit antibiotischer Beschichtung nach Anspruch 1, mit in Wasser gering löslichen Salzen aus der Gruppe des Netilmicin-Laurates, des Netilmicin-Myristates, des Netilmicin-Dodecylsulfates, des Sisomicin-Laurates, des Sisomicin-Myristates, des Sisomicin-Dodecylsulfates, des Amikacin-Laurates, des Amikacin-Myristates, des Amikacin-Dodecylsulfates, des Kanamycin-Laurates, des Kanamycin-Myristates, des Kanamycin-Dodecylsulfates, des Tobramycin-Laurates, des Tobramycin-Myristates, des Tobramycin-Dodecylsulfates, des Vancomycin-Laurates, des Vancomycin-Myristates, des Vancomycin-Dodecylsulfates, des Ciprofloxacin-Laurates, des Ciprofloxacin-Myristates und/oder des Clindamicin-Laurates.

## Claims

1. Porous medical implant body having an antibiotic coating, **characterized in that** a coating of at least one antibiotic salt sparingly soluble in water or in an aqueous medium and from the group consisting of netilmicin laurate, of netilmicin dodecylsulphate, of netilmicin myristate, of sisomicin laurate, of sisomicin myristate, of sisomicin dodecylsulphate, of gentamicin laurate, of gentamicin myristate, of clindamycin laurate, of amikacin laurate, of amikacin myristate, of amikacin dodecylsulphate, of kanamycin laurate, of kanamycin myristate, of kanamycin dodecylsulphate, of vancomycin laurate, of vancomycin dodecylsulphate, of vancomycin myristate, of tobramycin laurate, of tobramycin myristate, of tobramycin dodecylsulphate, of ciprofloxacin laurate and of ciprofloxacin myristate is introduced into the pore system of non-metallic porous bodies and/or of metallic porous bodies.

2. Process for the preparation of porous medical implant bodies having an antibiotic coating according to Claim 1, **characterized in that** first an aqueous solution which contains at least one member of a freely water-soluble salt of netilmicin, of sisomicin, of clindamycin, of amikacin, of kanamycin, of tobramycin, of vancomycin and of ciprofloxacin is introduced into the pore system of porous bodies, and **in that**, after a drying step, a second aqueous solution of a freely water-soluble salt of lauric acid, of myristic acid and/or of dodecylsulphuric acid is introduced and a sparingly water-soluble antibiotic coating is formed in the pore system of the porous body.

3. Process according to Claim 2, **characterized in that** the sequence of the coating steps is interchanged.

4. Process for the preparation of porous medical implant bodies having an antibiotic coating according to Claim 1, **characterized in that** a methanolic solution or an ethanolic solution of at least one member from the group consisting of netilmicin laurate, of netilmicin myristate, of netilmicin dodecylsulphate, of sisomicin laurate, of sisomicin myristate, of sisomicin dodecylsulphate, of gentamicin laurate, of gentamicin myristate, of clindamycin laurate, of tobramycin laurate, of tobramycin myristate, of tobramycin dodecylsulphate, of ciprofloxacin myristate, of vancomycin laurate, of vancomycin myristate and of vancomycin dodecylsulphate is introduced into the pore system of the porous body, and **in that** a sparingly water-soluble antibiotic coating is formed by vaporisation or evaporation of the methanol or of the ethanol.

5. Process for the preparation of porous medical implant bodies having an antibiotic coating according to Claim 1, **characterized in that** amikacin laurate, kanamycin laurate, amikacin dodecylsulphate and/or kanamycin dodecylsulphate is partially dissolved and/or suspended in a mixture of dioxane-water and/or tetrahydrofuran-water and these solutions and/or suspensions are introduced into the pore system of the porous body, and **in that** a sparingly water-soluble antibiotic coating is formed by vaporisation or evaporation of the mixtures of dioxane and water or of the mixtures of tetrahydrofuran and water.

6. Porous medical implant body having an antibiotic coating according to Claim 1, **characterized in that** the antibiotic coating is applied to porous bodies which are present in the form of porous powders, porous granules, porous mouldings and/or porous layers on compact bodies.

7. Porous medical implant body having an antibiotic coating according to Claim 1, **characterized in that** the coating of porous bodies which preferably have the form of porous powders and/or porous granules is formed by addition of at least one antibiotic salt sparingly soluble in water or in an aqueous medium and from the group consisting of netilmicin laurate, of netilmicin myristate, of netilmicin dodecylsulphate, of sisomicin laurate, of sisomicin myristate, of sisomicin dodecylsulphate, of gentamicin laurate, of gentamicin myristate, of clindamycin laurate, of amikacin laurate, of amikacin myristate, of amikacin dodecylsulphate, of kanamycin laurate, of kanamycin myristate, of kanamycin dodecylsulphate, of ciprofloxacin myristate, of tobramycin laurate, of tobramycin myristate, of tobramycin dodecylsulphate, of vancomycin laurate, of vancomycin myristate and of vancomycin dodecylsulphate, in particular by milling with addition of methanol, ethanol, dioxane, tetrahydrofuran and/or water or mixtures thereof.

8. Porous medical implant body having an antibiotic coating according to Claim 1, **characterized in that** the coating of porous bodies which preferably have the form of porous powders and/or porous granules is formed by addition of a mixture of at least one water-soluble salt of netilmicin, of sisomicin, of clindamycin, of amikacin, of kanamycin, of tobramycin, of vancomycin and/or of ciprofloxacin and at least one water-soluble salt of lauric acid, of myristic acid and/or of dodecylsulphuric acid in the presence of water or aqueous solutions, in particular by milling.

9. Porous medical implant body having an antibiotic coating according to Claim 1, **characterized in that** the coating optionally additionally contains freely water-soluble salts of gentamicin, of netilmicin, of sisomicin, of amikacin, of kanamycin, of clindamycin, of tobramycin, of vancomycin, of ciprofloxacin and/or of moxifloxacin.

10. Porous medical implant body having an antibiotic coating according to Claim 1, **characterized in that** the antibiotic coating is applied to absorbable, porous bodies, to partially absorbable porous bodies and/or to non-absorbable, biocompatible, porous bodies.

11. Use of porous medical implant bodies having an antibiotic coating according to Claim 1, which are in the form of antibiotically coated porous granules and/or antibiotically coated porous powders, as binders for the production of mouldings by compression moulding of powder mixtures.

12. Porous medical implant body having an antibiotic coating according to Claim 1, comprising sparingly water-soluble salts from the group consisting of netilmicin laurate, of netilmicin myristate, of netilmicin dodecylsulphate, of sisomicin laurate, of sisomicin myristate, of sisomicin dodecylsulphate, of amikacin laurate, of amikacin myristate, of amikacin dodecylsulphate, of kanamycin laurate, of kanamycin myristate, of kanamycin dodecylsulphate, of tobramycin laurate, of tobramycin myristate, of tobramycin dodecylsulphate, of vancomycin laurate, of vancomycin myristate, of vancomycin dodecylsulphate, of ciprofloxacin laurate, of ciprofloxacin myristate and/or of clindamycin laurate.

## Revendications

1. Implants médicaux poreux, avec revêtement antibiotique, **caractérisé en ce qu'**un revêtement consistant en au moins un sel d'un antibiotique faiblement soluble dans l'eau ou en milieu aqueux, choisi parmi le groupe du laurate de netilmicine, du dodéculsulfate de netilmicine, du myristate de netilmicine, du laurate de sisomicine, du myristate de sisomicine, du dodécylsulfate de sisomicine, du laurate de gentamicine, du myristate de gentamicine, du laurate de clindamycine, du laurate d'amikacine, du myristate d'amikacine, du dodécylsulfate d'amikacine, du laurate de kanamycine, du myristate de kanamycine, du dodécylsulfate de kanamycine, du laurate de vancomycine, du dodécylsulfate de vancomycine, du myristate de vancomycine, du laurate de tobramycine, du myristate de tobramycine, du dodécylsulfate de tobramycine, du laurate de ciprofloxacine, du myristate de ciprofloxacine, est introduit dans le système poreux de corps poreux non métalliques et/ou de corps poreux métalliques.

2. Procédé de préparation d'implants médicaux poreux, avec revêtement antibiotique, selon la revendication 1, **caractérisé en ce** l'on introduit d'abord dans le système poreux du corps poreux, une solution aqueuse qui contient au moins un représentant d'un sel facilement soluble dans l'eau de la netilmicine, de la sisomicine, de la clindamycine, de l'amikacine, de la kanamycine, de la tobramycine, de la vancomycine, de la ciprofloxacine, et en ce qu'après une étape de séchage, on introduit une deuxième solution aqueuse d'un sel facilement soluble dans l'eau de l'acide laurique, de l'acide myristique et/ou de l'acide dodécylsulfurique, et on forme ainsi, un revêtement antibiotique faiblement soluble dans l'eau, dans le système poreux du corps poreux.

3. Procédé selon la revendication 2, **caractérisé en ce que** la succession des étapes de revêtement est inversée.

4. Procédé de préparation d'implants médicaux poreux, avec revêtement antibiotique, selon la revendication 1, **caractérisé en ce** l'on introduit dans le système poreux du corps poreux, une solution méthanolique ou une solution éthanolique d'au moins un représentant du groupe du laurate de netilmicine, du myristate de netilmicine, du dodéculsulfate de netilmicine, du laurate de sisomicine, du myristate de sisomicine, du dodécylsulfate de sisomicine, du laurate de gentamicine, du myristate de gentamicine, du laurate de clindamycine, du laurate de tobramycine, du myristate de tobramycine, du dodécylsulfate de tobramycine, du myristate de ciprofloxacine, du laurate de vancomycine, du myristate de vancomycine et du dodécylsulfate de vancomycine, et en ce que par évaporation ou volatilisation du méthanol ou de l'éthanol, on forme un revêtement antibiotique faiblement soluble dans l'eau.

5. Procédé de préparation d'implants médicaux poreux, avec revêtement antibiotique, selon la revendication 1, **caractérisé en ce que** dans un mélange dioxanne-eau et/ou tétrahydrofuranne-eau, on dissout partiellement et/ou met en suspension, le laurate de d'amikacine, le laurate de kanamycine, le dodécylsulfate d'amikacine, et/ou le dodécylsulfate de kanamycine, **en ce que** l'on introduit cette solution et/ou suspension dans le système poreux du corps poreux, et **en ce que** par évaporation ou volatilisation du mélange de dioxanne et d'eau ou du mélange de tétrahydrofuranne et d'eau, on forme un revêtement antibiotique faiblement soluble dans l'eau.

6. Implants médicaux poreux, avec revêtement antibiotique, selon la revendication 1, **caractérisés en ce que** le revêtement antibiotique est appliqué sur le corps poreux, qui se présente sous forme de poudres poreuses, granulés poreux, corps moulés poreux et/ou couches poreuses sur des corps compacts.

7. Implants médicaux poreux, avec revêtement antibiotique, selon la revendication 1, **caractérisés en ce que** le revêtement des corps poreux, qui présentent de préférence, la forme de poudres poreuses et/ou de granulés poreux, est formé par addition d'au moins un sel d'un antibiotique faiblement soluble dans l'eau ou en milieu aqueux, choisi parmi le groupe du laurate de netilmicine, du myristate de netilmicine, du dodéculsulfate de netilmicine, du laurate de sisomicine, du myristate de sisomicine, du dodécylsulfate de sisomicine, du laurate de gentamicine, du myristate de gentamicine, du laurate de clindamycine, du laurate d'amikacine, du myristate d'amikacine, du dodécylsulfate d'amikacine, du laurate de kanamycine, du myristate de kanamycine, du dodécylsulfate de kanamycine, du myristate de ciprofloxacine, du laurate de tobramycine, du myristate de tobramycine, du dodécylsulfate de tobramycine, du laurate de vancomycine, du dodécylsulfate de vancomycine et du myristate de vancomycine, en particulier par broyage, et addition de méthanol, d'éthanol, de dioxanne, de tétrahydrofuranne, et/ou d'eau ou leur mélange.

8. Implants médicaux poreux, avec revêtement antibiotique, selon la revendication 1, **caractérisés en ce que** le revêtement des corps poreux qui présentent de préférence la forme de poudres poreuses et/ou de granulés poreux, est formé par addition d'un mélange d'au moins un sel soluble dans l'eau de la netilmicine, de la sisomicine, de la clindamycine, de l'amikacine, de la kanamycine, de la tobramycine, de la vancomycine, de la ciprofloxacine, et d'au moins un sel soluble dans l'eau de l'acide laurique, de l'acide myristique et/ou de l'acide dodécylsulfurique, en présence d'eau ou de solutions aqueuses, en particulier par broyage.

9. Implants médicaux poreux, avec revêtement antibiotique, selon la revendication 1, **caractérisés en ce que** le revêtement contient le cas échéant, en outre, des sels facilement solubles dans l'eau de la gentamicine, de la netilmicine, de la sisomicine, de l'amikacine, de la kanamycine, de la clindamycine, de la tobramycine, de la vancomycine, de la ciprofloxacine, et/ de la moxifloxacine.

10. Implants médicaux poreux, avec revêtement antibiotique, selon la revendication 1, **caractérisés en ce que** le revêtement antibiotique est appliqué sur un corps résorbable, poreux, sur un corps poreux partiellement résorbable et/ou sur un corps poreux, biocompatible, non résorbable.

11. Utilisation d'implants médicaux poreux, avec revêtement antibiotique, selon la revendication 1, qui sont formés de granulés poreux revêtus d'antibiotique et/ou de poudre poreuse revêtue d'antibiotique, comme liant pour la préparation de corps moulés, par compression de mélanges pulvérulents.

12. Implants médicaux poreux, avec revêtement antibiotique, selon la revendication 1, avec sels faiblement solubles dans l'eau du groupe du laurate de netilmicine, du myristate de netilmicine, du dodéculsulfate de netilmicine, du laurate de sisomicine, du myristate de sisomicine, du dodécylsulfate de sisomicine, du laurate d'amikacine, du myristate d'amikacine, du dodécylsulfate d'amikacine, du laurate de kanamycine, du myristate de kanamycine, du dodécylsulfate de kanamycine, du laurate de tobramycine, du myristate de tobramycine, du dodécylsulfate de tobramycine, du laurate de vancomycine, du dodécylsulfate de vancomycine, du laurate de ciprofloxacine, du myristate de ciprofloxacine et/ou du laurate de clindamicine.
